(19) Europäisches Patentamt
European Patent Office
Office européen des brevets

(11) **EP 0 579 630 B1**

(12) **EUROPEAN PATENT SPECIFICATION**

(45) Date of publication and mention
of the grant of the patent:
**05.01.2000 Bulletin 2000/01**

(51) Int Cl.[7]: **C12M 1/40**, C12M 1/12,
C12M 3/00, C12N 11/00,
C02F 3/00

(21) Application number: **92907241.1**

(22) Date of filing: **06.04.1992**

(86) International application number:
**PCT/EP92/00779**

(87) International publication number:
**WO 92/18609 (29.10.1992 Gazette 1992/27)**

(54) **MEMBRANES WITH IMMOBILIZED MICROORGANISMS THEREON AND/OR THEREIN, PROCESS FOR OBTAINING SUCH MEMBRANES, REACTOR COMPRISING SAID MEMBRANES AND PROCESS INVOLVING THE USE OF SAID MEMBRANES, IN PARTICULAR FOR THE ELIMINATION OF METALS OR THE DEGRADATION OF XENOBIOTIC ORGANIC COMPOUNDS**

Membranen mit immobilisierten Mikroorganismen darauf oder darin, Verfahren zur Herstellung solcher Membranen , Reaktor mit solchen Membranen und Verfahren unter Verwendung dieser Membranen, insbesondere zur Entfernung von Metallen oder zur Absetzung von organischen, xenobiotischen Verbindungen

MEMBRANES SUR LESQUELLES ET/OU DANS LESQUELLES SONT IMMOBILISES DES MICROORGANISMES, LEUR PROCEDE DE PRODUCTION, REACTEUR LES COMPORTANT, ET PROCEDE CONSISTANT A LES UTILISER, NOTAMMENT POUR L'ELIMINATION DE METAUX OU LA DEGRADATION DE COMPOSES ORGANIQUES XENOBIOTIQUES

(84) Designated Contracting States:
**AT BE CH DE DK ES FR GB GR IT LI LU MC NL SE**

(30) Priority: **12.04.1991 EP 91400986**

(43) Date of publication of application:
**26.01.1994 Bulletin 1994/04**

(73) Proprietor: **VITO**
**2400 Mol (BE)**

(72) Inventors:
• **DIELS, Ludo**
**B-2520 Oelegem (BE)**
• **LEYSEN, Roger**
**B-2400 Mol (BE)**
• **VAN ROY, Sandra**
**B-2450 Meerhout (BE)**
• **DOYEN, Willy**
**B-2160 Wommelgen (BE)**
• **MERGEAY, Maximilien**
**B-2470 Retie (BE)**

(74) Representative: **Van Malderen, Michel et al**
**Office van Malderen**
**Place Reine Fabiola 6/1**
**1083 Bruxelles (BE)**

(56) References cited:
**EP-A- 0 238 276**          **EP-A- 0 355 910**
**GB-A- 2 178 447**          **US-A- 3 883 393**
**US-A- 4 921 612**          **US-A- 4 988 443**

• **BIOTECHNOLOGY & BIOENGINEERING, vol. 36, no. 1, 5 June 1990, pages 97-103, New York, US, D. PATANKAR et al.: "Wall-Growth Hollow-Fiber Reactor for Tissue Culture: I. Preliminary Experiments", whole document**
• **PATENT ABSTRACTS OF JAPAN, vol. 12, no. 467 (C-550)(3314), 7 December 1988, & JP -A - 63188386 (SHIWADZU CORP) 03.08.1988, abstract**
• **BIOTECHNOLOGY & BIOENGINEERING, vol. 25, no. 11, November 1983, pages 2613-1681, New York, US, D.S. INCOES et al.: "Hollow-Fiber Membrane Bioreactors Using Immobilized E. coli for Protein Synthesis", whole article**

**Description**

The invention relates to membranes with immobilized microorganisms thereon and/or therein, to a process for obtaining such membranes, and to reactors comprising said membranes and to a process involving the use of said membranes, in particular for the elimination of metals or of xenobiotic organic compounds.

Soluble metal removal is a technical challenge which must be met before the recycling process water or avoiding toxic discharges in plant effluent.

Heavy metals such as cadmium, lead, copper and zinc draw the attention of the hygienists because of their toxicity. The public health is directly concerned with the occurrence of heavy metals in water and soil even at low concentrations owing to their accumulation in vegetables through soil solution. In the same way, sewage sludges produced in biological waste treatment can be also loaded with heavy metals and are often used as fertilizers. It is a tendency towards water reuse and rivers as reservoirs for drinking water. It is the reason why national and international authorities have issued directives on limit value for heavy metals in industrial effluents as well as in aquatic systems.

So, it appears desirable that these health hazards should be avoided. For this purpose, the best way should be the removal of heavy metals at the emission point i.e. in the industrial effluents.

The removal of heavy metals from industrial wastewaters can be performed by several strategies. Among them, chemical precipitation by addition of hydroxides or calcium oxides, ion-exchange on resin or electrolyses are of common practices. These methods are used when rather large amounts of metals, i.e. more than 500 ppm, are involved.

The use of ion exchange is more interesting at very low concentrations (less than about 5 ppm).

The disadvantages of ion exchange is high resin costs.

The disadvantages of electrolyses is high energy costs.

The disadvantages of hydroxides or calcium oxides is high sludge production.

However, they are not appropriate to remove intermediate amounts of metals or to degrade xenobiotic organic compounds.

It has also been investigated to use biomass which is immobilized in porous polysulfone beads for extracting toxic and heavy metals from dilute waste streams. The beads were fabricated from high-density polysulfone dissolved in dimethylformamide (DMF). Dried, thermally-killed biomass produced by algae, yeast, bacteria, and aquatic flora were blended into the polysulfone-DMF solution, and spherical beads were formed by injecting the mixture into water.

Contaminants removed from the waters included arsenic, cadmium, copper, mercury, lead, manganese, and zinc. Laboratory tests indicated that the beads may be especially useful in treating dilute wastewaters containing metal concentrations up to about 15 mg/l (cf. Jeffers T.H. et al., 1989, "Biosorption of metal contaminants using immobilized biomass", Biohydrometallurgy p. 317-327). This is a method applicable for very low concentrations.

In this case, it is necessary to regenerate the bacteria, because there is adsorption of the metals to be removed, on the sites of the bacteria, and hardly little precipitation and then the sites of the bacteria become saturated. When the bacteria have been regenerated, their efficiency to adsorb metal is lowered because all the sites cannot be regenerated or some sites are destroyed. No high upconcentration can be obtained.

It is necessary to use a large amount of beads, because it is possible to adsorb only between 5 and 10% of the metal with respect to the biomass, whereas if precipitation could take place (which is not the case), it would be possible to eliminate up to above 50% of metal with respect to the biomass.

U.S. Patent US-A-4 988 443 has disclosed the detoxification in an hollow fiber reactor of organic toxicants by microorganisms. The microorganisms are cultured in the shell side of the reactor with the cell adhering to the external surface of the tubular membrane. This system needs the use of an organic solvent to entrap the toxicants, and there is a risk to get microorganisms release since the cells adhere on the external surface and are unable to colonize the pores of the fibers.

Other reactors containing membranes have been developed. In EP-A- 0 355 910 the authors developed a reactor for bioconversion by microorganisms, for example for beer production, which contains a porous structure. The nature of the membrane does not allow their colonization without release of microorganisms. In Biotechnology and Bioengineering (26 1990, June 5, n° 1, p. 99), a hollow fiber reactor made of organic polymer was described; it allows the continuously growth of eucaryotic cells as hybridomas, but cannot be used for a process of metal recovering from industrial development or for degradation of xenobiotic organic compounds.

Debus O. et al. ("Aerobic mineralization of benzene, toluene and xylenes by microorganisms attached to gas-permeable membranes", Technical University of Hamburg-Harburg, FRG, April 1990) have disclosed the use of pure or mixed cultures of microorganisms to biodegrade volatile organics like benzene, toluene, ethylbenzene and the isomeric xylenes (BTEX), under aerobic conditions. In order to avoid the conventional aeration systems producing a large number of bubbles, leading to a BTEX loss by stripping, gas permeable membranes are used, such as silicon rubber. The loss of volatile organics can be minimized by allowing BTEX-mineralizing microorganisms to colonize the membrane surface and form a barrier to the escaping substances.

In this case, the bacteria receive only oxygen from the membranes. Moreover, the bacteria form a biofilm on the membranes, because the pores are too small for the bacteria to be immobilized on or in the membrane, and the nutrients are in the effluents, which involves a contamination of the effluents.

It is known that some microorganisms can immobilize metals up to high concentrations in their cellular materials specially when they are attached on a support. Among the culture devices which promote the growth of microorganisms on a surface, the biological fluidized bed is attractive because it has excellent adhesion potentialities for bacteria. The biological fluidized bed is composed of a cylinder packed with inert particles such as sand, anthracite, glass beads, plastic, stone gravels which provide support for microbial growth. The particles are freely suspended in the nutritive solution by an upward flow (cf. Remacle J. et al., Heidelberg 1983, "Uptake of heavy metals from industrial effluents by microorganisms developed in a biological fluidised bed" p. 936-939).

One of the drawbacks of this method is the fact that the nutritive medium for the microorganisms is mixed with the effluent to be treated, which involves a contamination of the effluent. Moreover, important amounts of carbon are needed, because they are added into the effluent. Besides, there is a big release of the microorganisms; both these factors increase the cost of the treatment of the effluent.

Reticulated polyurethane foams are interesting supports allowing a high retention and an easy recovery of biomass just by squeezing (Cooper P.F. et al., 1986, in Process Engineering Aspect of Immobilised Cell Systems p. 205-217 Webb C. et al. (eds), I. Chem. Eng.). These are essential characteristics for the development of a process of metal recovery from industrial effluents.

The immobilized cell cultures were conducted in fixed bed microfermenters (V = 0.41) continuously fed with a nutrient medium. The support particles consisted in 3.4 cm cubes with an internal porosity of 98% and a pore aperture of 30 ppi (Colombi Y. et al., 1987, "Cadmium uptake by Alcaligenes eutrophus immobilized in reticulated polyurethane foam", Proc. 4th European Congress on Biotechnology, 1: 120).

In this case, the biofilm is on the surface of the pores, which implies that there is a big release of cells. Besides, there is a need for important amounts of carbon.

During the last years, bacteria resistant to a variety of heavy metals were isolated and identified (Silver S. et al., 1988, "Plasmid-mediated heavy metal resistances" Ann. Rev. Microbiol. 42, 717-743). The mechanisms for these resistances are often controlled by plasmid borne genes or by transposons. A remarkable example of those resistant bacteria is Alcaligenes eutrophus var. metallotolerans. The representative strain CH34 was isolated in sediments from a decantation basin of a zinc factory (Mergeay M. et al., 1978, "Extrachromosomal inheritance controlling resistance to cadmium, cobalt and zinc ions: evidence from curing in a Pseudomonas" Arch. Int. Physiol. Biochim. 86, 440-441). Strain CH34 bears two large plasmids (Mergeay M. et al., 1985, "Alcaligenes eutrophus CH34 is a facultative chemolithotroph with plasmid-bound resistance to heavy metals". J. Bacteriol. 162, 328-334) controlling resistance against $Cd^{++}$, $Co^{++}$, $Zn^{++}$, $Hg^{++}$, $Tl^+$, $Cu^{++}$, $Pb^{++}$ (pMOL30, 240 kb) and $Co^{++}$, $Zn^{++}$, $Ni^{++}$, $Hg^{++}$, $CrO_4^{--}$, $Tl^+$ (pMOL28, 165 kb). On pMOL28 nickel, cobalt and zinc genes (cnr) are probably on the same cluster very near to the chromate genes (chr). One DNA fragment (in pMOL30) of about 9 kb (Nies D. et al., 1987, "Cloning of plasmid genes encoding resistance to cadmium, zinc and cobalt in Alcaligenes eutrophus" Bacteriol. 169, 4865-4868) is responsible for the resistance against cadmium, zinc and cobalt (czc). And another gene cluster seems to code for copper and lead resistance (cup). Both plasmids contain a mercury transposon : Tn4378 and Tn4380 (Diels L. et al., 1985, "Mercury transposons from plasmids governing multiple resistance to heavy metals in Alcaligenes eutrophus CH34", Arch. Int. Physiol. Biochim. 93, B27-B28 ; Diels L. et al., 1989, "Large plasmids governing multiple resistances to heavy metals : a genetic approach" Toxic. Environm. Chem. 23, 79-89). Different heavy metal resistances genes are cloned and sequenced, namely czc (Nies D.H. et al., 1989, "Expression and nucleotide sequence of a plasmid determined divalent cation efflux system from Alcaligenes eutrophus", Proc. Natl. Acad. Sci. USA 86, 7351-7356), cnr (Siddiqui R.A. et al., 1989, "Cloning of pMOL28-encoded nickel resistance genes and expression of the gene in Alcaligenes eutrophus and Pseudomonas spp", J. Bacteriol. 171, 5071-5078) and chr (Nies A. et al., 1990, "Nucleotide sequence of chr genes responsible for resistance to chromate in Alcaligenes eutrophus", J. Biol. Chem. 265, 5648-5653). From the copper and lead genes, mutants are available.

The czc and mercury genes were used as probes for hybridization with total DNA from strains isolated from different mining and industrial sites in Belgium and Zaire (Diels L. et al., 1988, "Isolation and characterization of resistant bacteria to heavy metals from mining areas of Zaire", Arch. Int. Physiol. Biochim. 96, B13 ; Diels L. et al., 1988, "Detection of heterotrophic bacteria with plasmid-bound resistances to heavy metals from belgian industrial sites", Arch. Int. Physiol. Biochim. 96, B14). From these different sites, strains hybridizing with these probes could be isolated (Diels L. et al., 1990, "DNA probe-mediated detection of resistant bacteria from soils highly polluted by heavy metals" Appl. Environm. Microbiol. 56, 1485-1491).

As could be shown by Nies D. et al. (1989, "Plasmid determined inducible efflux is responsible for resistance to cadmium, cobalt and zinc in Alcaligenes eutrophus" J. Bacteriol. 171, 896-900 and 1989, "Metal ion uptake by a plasmid-free metal sensitive Alcaligenes eutrophus strain", J. Bacteriol. 171, 4073-4075) resistance to chromate is inducible and based on decreased net accumulation of the metal anion. Resistance to zinc, cadmium, cobalt and nickel are resulting from inducible, energy dependent cation efflux systems. In some physiological circumstances Alcaligenes eutrophus can also accumulate and precipitate heavy metals (Diels L., 1990, "Accumulation and precipitation of Cd and Zn ions by Alcaligenes eutrophus strains", Biohydrometallurgy (1989) 369-377 ; Diels L. et al., 1989, "Isolation and identification of bacteria living in environments severely contaminated with heavy metals", 7th International Conference on Heavy Metals in the Environment, 12-15 September 1989, Geneva). At increased concentrations of Cd or Zn ions, a removal of these metals from the solution is observed during the late log phase and the stationary phase. This accumulation and precipitation is correlated with the concentration and kind of carbon source (lactate or gluconate), with the progressive alkalinization of the periplasmic space and the surrounding medium, due to the proton antiporter system of the resistance mechanism, with the concentration of phosphate and appears to be associated with the outer cell membrane. The precipitation of $CdCO_3$ and $Cd(OH)_2$ is proved by IR-spectroscopy. The interpretation of this feature is that the metal speciation will change at the cell surface due to the progressive pH increase, the steep pH gradient on this site, and the production of $CO_2$ by the cell metabolism.

For electrochemical purposes, membranes have been developed (Leysen R. et al., 1987, "The use of heterogeneous membranes in electrochemical systems", in "Synthetic polymeric membranes"", Eds. B. Sedlacek and J. Kahovec, W. de Gruyter, Berlin) composed of a polymeric polysulfone material in which zirconium oxide grains are distributed in a homogeneous way in order to form a composite membrane ; these membranes are formed using the phase inversion technique (evaporation-crystallization).

This type of membrane has already been produced in three different configurations : flat membranes (Doyen W. et al., 1988, "The use of $ZrO_2$-based composite membranes for the separation of oil-water emulsions", in Proceedings of the Symposium on "Particle Technology in relation to Filtration Separation", Antwerp, October 3-4) with or without a reinforcing support, hollow fibers (Matthys J. et al., 1989, "Development of hollow fibers for the production of secretory products by living cells, in Proceedings of the Symposium on "Down stream processing in Biotechnology", Bruges, April 10-11) and tubes (Doyen W. et al., 1989, "New composite tubular membranes for ultrafiltration" in Proceedings of the "6th International Symposium on Synthetic Membranes in Science and Industry", Tübingen, September 4-8); however, the hollow fibers (when their diameter is smaller than about 4 mm) are very sensitive to clogging especially in the presence of an effluent which contains suspended materials.

An aspect of the invention is to provide with membranes with immobilized microorganisms thereon and/or therein, enabling to eliminate low metal concentrations, particularly of heavy metals.

Another aspect of the invention is to provide with membranes with immobilized microorganisms thereon and/or therein, enabling to degrade xenobiotic organic compounds.

Another aspect of the invention is to provide with membranes with immobilized microorganisms thereon and/or therein, enabling to synthesize compounds when the microorganisms contain the appropriate cellular machinery.

Another aspect of the invention is to provide with membranes with immobilized microorganisms thereon and/or therein, enabling to separate the effluent to be treated from the nutrient medium necessary for the life of microorganisms, i.e. enabling to recover a non contaminated effluent.

Another aspect of the invention is to provide with reactors comprising membranes with immobilized microorganisms thereon and/or therein enabling to treat effluents contaminated with metals, said effluents being separated from the nutrient medium necessary for the life of the microorganisms.

Another aspect of the invention is to provide with reactors having both a good metal removal capacity and a reduced need for the concentration of nutrients.

A membrane with immobilized microorganisms thereon and/or therein according to the invention is characterized in that

- said microorganisms are alive or in a viable form and are liable to precipitate one or several metals, when they are in the presence of said metal and/or to degrade xenobiotic organic compounds, when they are in the presence of said compounds, and in that
- said membrane is of a porous material, being either an inorganic oxide or a composite material containing an inorganic oxide, the membrane parameters being such that the microorganisms can settle in the pores, which are communicating between themselves, and which allow an appropriate colonization of said membrane by said microorganisms, and that their release is less than 104 microorganisms/ml/h, preferably less than $10^2$ microorganisms/ml/h.

The object of the invention is defined in the claims.

By "microorganisms", one may cite, for instance, bacteria, algae, yeasts, fungi.

Bacteria are advantageously used for the membranes of the invention.

An important feature of the membranes of the invention is the fact that the size of the pores is appropriate to the size of the microorganism which is immobilized in the membrane, i.e. large enough for the microorganisms to settle and to grow and not too large to prevent the release of said microorganisms, especially in the effluent to be treated.

The membranes with immobilized microorganisms, especially bacteria, thereon and/or therein according to the invention can be used to remove metals if the bacteria can induce the accumulation and/or the precipitation of said metals when the latter are in a solution (hereafter called "effluent"), when said effluent is in the presence of said membranes.

Accumulation corresponds to the adsorption of the metals on the sites of the bacteria, said sites being on the bacteria walls or in the extracellular polymers of said bacteria walls.

The precipitation of the metal takes place on the bacteria walls, as well as on the membranes and in the vessel in which the membranes can be located.

By metal, one designates the transition metals, the rare earth, the elements having metallic properties in the families IIIa, IVa, Va and VIa of the Mendelieff table.

By metals, one may cite for example cadmium, zinc, cobalt, copper, lead, mercury, thallium, chromium and manganese under the form of salts, either in a soluble or non soluble state.

The membranes with immobilized bacteria thereon and/or therein can also be used to degrade xenobiotic organic compounds, if the microorganisms have the genes responsible for the degradation or mineralization of these compounds and if they can express them.

The xenobiotic organic compounds designate the compounds which may endanger health and which are man made chemicals (non naturally occurring compounds). By way of example, one may cite fungicides, herbicides, pesticides, insecticides, chloroorganic compounds, particularly biphenyl compounds.

Advantageously, the membranes of the invention are used to eliminate metals and/or to degrade xenobiotic organic compounds.

The membranes contain pores at its surface and in its thickness in which the microorganisms can nest and grow, which implies that the pores must be large enough for the size of the microorganisms and must not be too large otherwise the microorganisms would be released from the pores.

The expression immobilized microorganisms and/or in the membrane means that the microorganisms are trapped in the pores of the membrane, but can migrate from one pore to another one, especially if space is needed after cellular division.

When the membrane has immobilized microorganisms thereon and/or therein, the microorganisms can migrate in the direction of the nutrient medium in the presence of which the membrane can be.

The expression "appropriate colonization" means that, after the incorporation of the bacteria in the pores of the membrane and due to the growth of the bacteria, the pores of the membranes can be filled by bacteria in a sufficient amount for the process to be efficient. An efficient process is a process in which the bacteria can grow on the membrane and exert their function at the outside of the membrane (being the effluent side).

The microorganisms on and/or in the membranes must be non killed, i.e. alive or liable to be brought back to life, for instance lyophilized, in so far as the lyophilization process does not destroy most of the microorganisms.

The membranes must be such that the release of bacteria is less than about $1 \times 10^4$ cells/ml/h, preferably less than about $1 \times 10^2$ cells/ml/h.

For heavy metal removal a direct contact between the metals and the bacteria is necessary to induce metal precipitation and crystallisation.

When there is no cell release from the membrane, the metal moves to the immobilized cells and precipitates around them. This results in crystal growth in and on the membrane. With this system heavy metal removal is also very slowly. And once the membrane is saturated an acid treatment is necessary for regeneration of the membrane, but with the risk of eliminating all viable immobilized cells.

When there is indeed cell release, the metals come immediately in contact with the bacteria around which they cristallize. This is a faster process and gives no membrane regeneration problems. Besides, the bacteria with crystallized metals must be removed by a recuperation column (such as a glass bead column). When no more metal is available, the free bacteria will bind with much difficulty to the recuperation column; this means that cell release must be steered in function of metal concentration. The cell release can be influenced by the diameter of the skin pores of the membrane or by the pump flow rate in the effluent at the membrane.

If the release is above these values, there is a contamination of the effluent containing the metal or organic compound to be eliminated.

The membranes of the invention can enable to eliminate up to 90% of metals contained in an effluent to be treated or up to 90% of xenobiotic organic compounds contained in an effluent to be treated.

An advantageous group of membranes with immobilized bacteria thereon and/or therein is constituted by the membranes in which the pores of the membranes have a maximum size of about 1 μ at the skin side (hereafter defined) and between about 1 μ and about 5 μ at the other side.

An advantageous group of membranes with immobilized bacteria thereon and/or therein of the invention is constituted by membranes wherein the porosity is of about 50% to about 80%, preferably of about 65% to about 75%.

The porosity is defined by the ratio between the volume of the pores and the volume of the total material which constitutes the membranes.

The thickness of the membranes must advantageously be such that there is appropriate tightness between the two parts located respectively on each of the sides of the membrane, said parts being created by the division of the space, by the membranes.

When the membranes are thicker (i.e. more than about 200 μ), the surface of the membranes can be smaller, for the same results of elimination of metals or xenobiotic organic compounds.

Moreover, in the case of thicker membranes, i. e. of more than about 200 μ, the release of the bacteria is smaller. To give an idea, a thickness of about 250 μ reduces the release of microorganisms of about 10 times with respect to a membrane of about 60 μ.

Besides, when the membranes are thicker, i.e. more than about 200 μ, there is a good accumulation of the metal on the membrane. To give an idea, a thickness of about 250 μ enables to increase the accumulation of metal of about 5 times with respect to a membrane of about 60 μ.

The thickness of the membranes is an important aspect, especially when the metal concentration is low.

In fact, when the metal concentration is low, i. e. of less than about 1 ppm, the precipitation does not work very well, and the fact that the metal can accumulate on the bacterial wall plays an important part in the efficiency of the metal removal.

To give an idea, 50% of a metal can be eliminated from a solution containing 0,5 ppm of said metal.

Advantageously, the membranes with immobilized microorganisms, particularly bacteria, thereon and/or therein are such that the thickness of the membranes is of about 50 μ to about 700 μ, preferably of about 70 to 500 μ, and more advantageously of about 250 μ to about 500 μ.

Preferably, the membranes with immobilized microorganisms, especially bacteria, thereon and/or therein according to the invention are substantially homogeneous in the whole of their thickness.

Said homogeneity corresponds both to the fact that the pores are substantially equally distributed in the membrane and to the fact that the size of the pores is substantially the same.

It is advantageous that the membrane should not contain large pores or finger like structures (i.e. pores of over 10 μ), to prevent an important release of bacteria.

It is also advantageous that the pores are not finger like shaped, because the trapping of microorganisms is not good, and consequently, release can be enhanced.

According to an advantageous embodiment of the invention, one of the sides of the membrane form a skin having pores, the size of which prevents the bacteria from being released, the size of these pores being advantageously smaller than about 1 μ, and preferably smaller than about 0.5 μ.

The side of the membrane which contains the smaller pores is hereafter named the "skin side".

According to another advantageous embodiment of the invention, on one of the sides of the membranes, externally, the bacteria are in the form of a biofilm of a thickness of about 1 to about 50 μ, preferably of about 10 to about 20 μ and internally the bacteria form a colonizing front, around single dispersed bacteria in the membrane.

The biofilm is always located externally on one of the sides of the membranes, but it is not always present.

The biofilm can be defined as a mass of bacteria which are fixed to each other by their own polymers.

The biofilm results from the growth of the bacteria, which have previously been introduced into some pores on the surface of said membrane.

The colonizing front is always present in the membranes and it corresponds to the filling of the pores by the bacteria, said colonizing front extends from one side of the membrane and have a thickness of about 10 μ, preferably 50 μ, to about the totality of the thickness of the membrane.

The growth of the microorganisms can be controlled by the nutrient medium and by the supply of oxygen.

After being introduced into some of the pores of the membrane, in the membrane and on the surface of the membrane, the bacteria grow inside the membrane, and on its surface, provided the size of the pores enables it and provided the pores communicate between themselves.

When the membrane comprises a skin side, the biofilm is opposite to the skin side.

As to the colonizing front, it is inside the membrane and limited to the membrane thickness.

To give an idea, when the growth of the bacteria on the membrane has been monitored such that the biofilm is opposite to the skin side, outside the membrane, the colonizing front can have a thickness of about 10 μ, preferably 50 μ, to about the totality of the thickness of the membrane, and preferably of about 100 μ to about 200 μ.

The shape of the membrane must be such that it forms a surface liable to be a separation for two different parts located respectively on each side of the membrane.

Advantageously, the surface of the membrane is flat or tubular, with or without a support.

When the membrane is flat, its size can be of about 10 $cm^2$ to about 1 $m^2$.

When the membrane is flat, it is advantageously combined with a support which prevents the membrane from being torn in case the membrane is subjected to high pressures, or to pressure differences between the two parts located respectively on each side of the membrane.

It is advantageous to combine a support on either side of the membrane, or on both sides of the membrane, preferably perpendicular to the direction of the pression.

Said support has substantially the surface size of the membrane and has a thickness of about 100 $\mu$ to about 1000 $\mu$, and is provided with holes of about 50 $\mu$ to about 500 $\mu$, preferably of about 100 $\mu$.

Said support is advantageously adjacent to the membrane.

Said support is advantageously constituted of the following material : polyester with a pore diameter between about 10 $\mu$ to about 200 $\mu$.

When the membrane surface is flat, there is advantageously a biofilm of microorganisms, particularly bacteria located on the external side of the membrane which has the larger pore size, i.e. opposite the skin side.

When the membrane surface is tubular, the colonizing front of bacteria is necessarily on the internal side of the membrane, and can move up to the internal part of the skin side.

Advantageously, when the membrane is tubular, a colonizing front is close to the internal side of the membrane which has the smaller pore size.

The tubular membrane advantageously is supported by a tube, and is located either on the external surface of said tube or on the inner surface of said tube.

The tubes used are porous tubes, preferably carbon tubes or polyester tubes.

When the membranes are tubular and coat the inner surface of the tube and have a skin side, the larger size pores are preferably in contact with the inner surface of the tube and the colonizing front is preferably close to the skin side, inside the membrane.

When the tubular membrane is not supported by a tube, it is called a hollow fiber membrane. For instance, the length is from about 30 to about 100 cm, the diameter is from about 5 to about 10 cm.

Advantageously, the membrane is made of polysulfone comprising an inorganic material, such as $ZrO_2$.

The membranes used are for instance described in European Patent Application n° 241995.

Pore formers such as $Sb_2O_5$, $CaCO_3$ or polyvinylpyrrolidone (PVP) can be added during the manufacture of the membrane for the production of pores, but are eliminated at the end of the production process of the membrane.

Preferred membranes have the following composition:

about 80% $ZrO_2$,
about 20% polysulfone.

Other preferred membranes are made of inorganic materials, such as inorganic oxides, such as $ZrO_2$ or $Al_2O_3$.

In these cases, the material of the membranes is designated by ceramics.

Advantageously, the microorganisms are liable to precipitate one or several metals, when they are in the presence of said metal and/or to degrade xenobiotic organic compounds, when they are in the presence of said compounds, or to synthesize compounds when a substrate to be transformed is present.

Advantageous microorganisms used are bacteria.

An advantageous group of bacteria is Alcaligenes eutrophus CH34 (ATCC N° 43123), which can be used to precipitate one or several heavy metals such as zinc, cadmium, nickel, lead and copper.

Another advantageous bacteria is A5 (deposited at the C.N.C.M., Institut Pasteur, 28 rue du Dr Roux, 75015 Paris, on February 28, 1991, under N° 1-1047), liable to degrade xenobiotic organic compounds.

The membranes of the invention enable to remove up to about 225 ppm of cadmium, up to about 720 ppm of zinc, up to about 150 ppm of nickel up to about 150 ppm of lead, and up to about 60 ppm of copper. The lowest concentrations which can be removed are at least in the order of 1 ppm.

The invention also relates to membranes with immobilized bacteria according to the invention, wherein the pressure difference between each side of the membrane is about 0.

The pressure difference over the membrane will be defined as :

$$\Delta P = P2 - P1$$

in which

P1 = pressure in effluent
P2 = pressure in nutrient

When the reactor will be loaded (immobilization) with bacteria, $\Delta P$ will be slightly larger than zero in order to suck the bacteria into the membrane. During the growth of the bacteria in the membrane (colonization process) $\Delta P$ will be zero. For heavy metal removal, $\Delta P$ can be zero or slightly positive and for xenobiotic degradation, $\Delta P$ can be zero or slightly negative, in order to eliminate cell release in this case.

The preparation of the membranes with the immobilized microorganisms thereon and/or therein, can be carried out as follows :

- the filtration of a suspension of microorganisms, containing an amount of microorganisms such that the membrane is not clogged and containing appropriate amounts of microorganisms, said suspension containing advantageously $10^8$ to $10^9$ microorganisms/ml, preferably $10^8$ microorganisms/ml, the filtration being carried out through the membrane, preferably in a tangential manner with respect to the surface of the membrane, in order to immobilize the microorganisms in and/or on the membrane.

The preparation of the membranes with the immobilized bacteria thereon and/or therein, can be carried out as follows :

- the filtration of a suspension of bacteria, containing an amount of bacteria such that the membrane is not clogged and containing an appropriate amount of bacteria, said suspension containing advantageously $10^7$ to $10^8$ bacteria/ml, preferably $10^8$ bacteria/ml, the filtration being carried out through the membrane, preferably in a tangential manner with respect to the surface of the membrane, in order to immobilize the bacteria in and/or on the membrane.

If the filtration is tangential with respect to the membrane, the bacteria can settle in the pores or if not are released in the suspension, but clogging of the membrane is avoided.

If the filtration is perpendicular with respect to the membrane, bacteria settle upon each other and clogging appears.

The bacteria used are preferably constituted by an overnight culture ($10^9$ bacteria/ml), which is preferably about 10 times diluted, to avoid clogging.

If the suspension contains at least $10^9$ bacteria/ml, there is a risk of clogging of the membrane.

It is possible to use less than $10^7$ bacteria/ml, but under these conditions, it would be necessary to wait until enough bacteria have grown, otherwise the metal or xenobiotic elimination would last too long.

In the process for immobilizing the microorganisms on the membrane, it is advantageous to colonize the immobilized microorganisms, until the pores of the membrane are filled by the microorganisms on a thickness of about 100 $\mu$ to about 200 $\mu$, preferably in the whole thickness of the membrane, in the presence of an appropriate nutrient medium.

The colonization corresponds to the growth of the microorganisms inside the membrane, and also possibly on the membrane until a biofilm is obtained.

The colonization until the pores of the membrane are completely filled lasts advantageously 4 days.

The advantage of colonization is that there is a high concentration of bacteria and the treatment process is more efficient.

To prepare the membranes of the invention, the microorganisms are immobilized on the membranes after the preparation of the membranes, to be sure that the microorganisms are microorganisms trapped in the pores and not in the mass of the material of the membranes.

To prepare tubular membranes, resort may be had to the process described in European Patent Application n°241995.

To prepare tubular membranes coated inside tubes or outside tubes, it is possible to resort to process known by the man skilled in the art, such as bringing a suspension of the components of the membrane to the shape of a hollow tube by means of a casting bob.

The membranes with bacteria immobilized thereon and/or therein are advantageously used in new reactors.

Said reactors of the invention comprise a recipient (cell) containing :

- a membrane with immobilized microorganisms thereon and/or therein, said microorganisms being alive or in a viable form, said membrane being of a porous material such that the microorganisms can settle in the pores, said pores communicating between themselves so that it makes it possible an appropriate colonization of the membrane by the microorganisms, and such that the release of microorganisms is not significant,
- possibly a support,
- two parts with each an inlet and an outlet and separated by a membrane, one of the two parts being in contact with one side of the membranes, and containing a nutrient medium to enable the life and growth of the microorganisms, and the other one, being in contact with the other side of the membranes and containing an effluent to be treated.

"Not significant" means that there is advantageously a release of less than about $10^4$ microorganisms/ml/h and preferably less than about $10^2$ microorganisms/ml/h.

The reactors of the invention are new, not only because of the membranes used, but also because they correspond to a new configuration, in so far as these reactors comprise two parts corresponding respectively to the effluent to be treated and to the nutrient medium, said two parts being created in the cell of the reactor by the separation of said cell with an appropriate membrane.

Said reactors of the invention comprise a recipient (cell) containing :

- a membrane with immobilized bacteria thereon and/or therein, said bacteria being alive or in a viable form, said membrane being of a porous material such that the bacteria can settle in the pores, said pores communicating between themselves so that it makes it possible an appropriate colonization of the membrane by the bacteria, and such that the release of bacteria is less than about $1.10^4$ bacteria/ml/h, preferably than about $1.10^2$ bacteria/ml/h,
- possibly a support,
- two parts with each an inlet and an outlet and separated by a membrane, one of the two parts being in contact with one side of the membranes, and containing a nutrient medium to enable the life and growth of the bacteria, and the other one, being in contact with the other side of the membranes and containing an effluent to be treated.

Said reactor can comprise anyone of the membranes of the invention above defined.

The parts hereto above-mentioned will be designated by "chambers".

The bacteria, immobilized on a membrane, receive the nutrient by diffusion of said nutrient medium through the membrane.

It is necessary to bring oxygen to the membrane to promote the development of the abovesaid biofilm.

Preferably, oxygen is brought to the membrane on the effluent side.

Preferably, there is no oxygen in the nutrient side.

The maximum amount of oxygen corresponds to saturation (i.e. about 10 mg of oxygen per liter).

The conditions in the reactor are such that there is growth of the microorganisms inside the membrane and not too much growth of the biofilm.

When the thickness of the biofilm is above about 100 $\mu$ bacteria can be released from the biofilm and this is preferably avoided, because it is a loss of bacteria. If the biofilm is on the nutrient medium side, then there is a loss of nutrient medium, because of the released bacteria. If the biofilm is on the effluent side, there is a contamination of the effluent medium by the released bacteria.

Moreover, when the thickness of the biofilm is above about 100 $\mu$, the bacteria which are close to the membrane are under anaerobic conditions and inefficient.

The effluent to be treated is advantageously a continuous flow.

The conditions in the reactor above referred to are :

- the composition of the nutrient medium,
- the amount of the nutrient medium,
- the amount of oxygen,
- the ratio of nutrient volume to effluent volume,
- the flow rate of the effluent volume and
- the flow rate of the nutrient medium.

The flow rate of the effluent medium must not be too high so that too many microorganisms are not released into the effluent medium.

An advantageous flow rate of the effluent medium is of about 1 ml/mn/cm$^2$ to about 4 ml/mn/cm$^2$, preferably of about 2 ml/mn/cm$^2$.

As to the nutrient medium, it can be or not continuous and its flow rate can preferably vary from about 10 $\mu$l/mn/cm$^2$ to about 200 $\mu$l/mn/cm$^2$, preferably of about 0 to about 100 $\mu$l/mn/cm$^2$.

An advantageous ratio between the volume of nutrient medium with respect to the volume of the effluent is about 1/10 to about 1/1000.

By way of example, in a reactor of the invention, the effluent volume is about 5 1 and the nutrient medium volume is of about 500 ml, for a surface membrane of about 10 cm$^2$.

The effluent must have a pH such that it does not kill the microorganisms.

The effluent must be previously treated so that the pH is about 6 to about 8, preferably about 7 to about 7.5.

The effluent must contain no organic solvent in an amount liable either to kill the microorganisms or to dissolve the membrane. The organic solvent, if any, must be under about 5% (expressed in weight).

In the nutrient medium, it must be taken into account the fact that the immobilized microorganisms need less nutrient elements than when they are free.

The amount of the nutrient medium needed for the immobilized bacteria is about 10 times less than the amount of nutrient medium needed for the free microorganisms.

The nutrient medium comprises :

- a carbon source,
- a phosphate,
- a nitrogen.

As a carbon source, one may use lactate or acetate.

Lactate is advantageously used, in an amount of about 1 g/l for 10$^9$ bacteria/ml, i.e. about 0,1 g/l for 10$^8$ bacteria/ml. This corresponds to about 0.3 g/l of effluent.

The phosphate used is advantageously under the form Na$_2$HPO$_4$.

It is used in a concentration of about 1 mM for 10$^9$ bacteria/ml, i.e. 0.1 mM for 10$^8$ bacteria/ml. This corresponds to about 0.02 mM/l of effluent.

The nitrogen used is advantageously under the form NH$_4$OH.

It is used in a concentration of about 1 mM for 10$^9$ bacteria/ml, i.e. 0.1 mM for 10$^8$ bacteria/ml. This corresponds to about 0.2 mM/l of effluent.

The pH of the nutrient medium is advantageously from about 7.5 to about 9, and is advantageously about 7,8.

An advantageous reactor of the invention comprises a flat membrane with immobilized microorganisms, especially bacteria, thereon and/or therein according to the invention, said membrane separating two chambers with each an inlet and an outlet, one of the chambers containing a nutrient medium and the other chamber containing an effluent to be treated.

The chamber containing the nutrient medium can be either in contact with the side of the membrane with the smaller pores or can be in contact with the side of the membrane with the larger pores.

When there is a biofilm, it can be either in contact with the effluent or in contact with the nutrient medium.

Advantageously, the chamber containing the effluent medium is in contact with the skin side of the membrane and the chamber containing the nutrient is in contact with the side of the membrane with the larger pores.

Advantageously, the biofilm of bacteria is located externally on the side of the membrane with the smaller pores, and the biofilm is in contact with the effluent.

In this case, the bacteria are immobilized, preferably by tangential filtration of a suspension of bacteria introduced into one of the chambers.

The suspension of bacteria must be introduced into the chamber which is in contact with the side of the membrane with larger pores, i.e. opposite to the skin side.

The suspension of bacteria is introduced either into the chamber which is destined to receive the effluent, after immobilization of the bacteria on the membrane or into the chamber which is destined to receive, after immobilization the nutrient medium, depending on the orientation of the skin side.

During the immobilization, it is not necessary that the bacteria be in the presence of a nutrient medium.

When the immobilization of the bacteria is over, colonization is carried out in the presence of a nutrient medium which diffuses to the bacteria immobilized. When the colonization is over, the effluent to be treated is introduced into one of the chambers to receive it.

In one chamber nutrient solution is pumped during reactor use and at the other side an effluent solution is pumped. With this system a reduction of nutrients can be obtained.

Bacteria immobilized on and/or in the membrane are fed by nutrients coming from the nutrient side. At the effluent side the immobilized microorganisms remove the heavy metals from the effluent. Siphons are used to keep the pressure difference over the membrane at zero.

The leakage between the nutrient medium and the effluent is prevented in so far as the amount of nutrient medium is such that it is taken up by the microorganisms immobilized in and/or on the membrane.

The leakage between the effluent and the nutrient medium can be avoided by keeping in a constant way, low concentrations of nutrients, in the nutrient solution.

Another advantageous reactor of the invention comprises :

- a tubular recipient, said recipient containing hollow tubes of a porous material, preferably carbon tubes, the inner surface or the outer surface of the carbon tubes being coated with a membrane according to the invention, said membrane being in contact with the effluent to be treated, the thickness of the carbon tubes being

- such that the microorganisms can be immobilized in the membranes by filtration of a suspension of microorganisms substantially along the outer or the inner surface of the tubes, and

- such that the nutrient medium which is either in the tube or in the intertubular space can diffuse from the outer or the inner surface of the tube respectively to the inner or to the outer surface of the tube coated with the abovesaid membrane.

In this embodiment, the membrane with immobilized microorganisms, especially bacteria, thereon and/or therein can be on the outer surface of the tubes.

In these conditions, the side of the membrane having the skin side is preferably opposite to the side of the membrane which is in contact with the outer surface of the membrane.

In this embodiment, the effluent to be treated is preferably in the intertubular space and the nutrient medium is inside the tube.

In this case, the microorganisms have been immobilized, preferably by tangential filtration, along the inner surface of the tube, of a suspension of bacteria.

Advantageously, the membrane with immobilized bacteria thereon and/or therein coat the inner surface of the membrane. In these conditions, the side of the membrane having the skin side is preferably opposite to the side of the membrane which is in contact with the inner surface of the tube.

In this embodiment, the effluent to be treated is preferably inside the tube and the nutrient medium is in the intertubular space.

In this case, the bacteria have been immobilized preferably by filtration, along the outer surface of the tube, of a suspension of bacteria.

Another advantageous reactor of the invention comprises :

- a tubular recipient, said recipient containing hollow tubes (supporting tubes) of a porous material, said supporting tubes being carbon tubes or polyester tubes, the inner surface of the supporting tubes being coated with a membrane according to the invention, said membrane being in contact with the effluent to be treated, the thickness of the carbon tubes being

- such that the microorganisms can be immobilized in the membranes by filtration of a suspension of microorganisms substantially along the outer surface of the tubes, and

- such that the nutrient medium which is in the intertubular space can diffuse from the outer surface of the tube respectively to the inner surface of the tube coated with the abovesaid membrane.

Preferably, the thickness of the carbon tube is about 1 to about 3 mm, and the thickness of the polyester tubes is about 100 to about 500 μ.

In this embodiment, the bacteria are first immobilized by a filtration of a suspension of bacteria tangentially along the outer surface of the tube, said bacteria going through the supporting tube and settling in and/or on the membrane. Then, the introduction of a nutrient medium in the intertubular space, which diffuses through the tubes to the membrane allow the bacteria to grow up to the skin side, forming thus a colonizing front close to the skin side.

Then the effluent to be treated can be introduced into the inner surface of the membrane, through the tube.

The nutrient medium can also be inside the inner surface of the membrane and the effluent medium can also be in the intertubular space.

The invention also relates to a reactor according to the invention, comprising

- a tubular recipient, said recipient containing hollow tubes (supporting tubes) of a porous material, said supporting tubes being carbon or polyester tubes, the inner surface of the supporting tubes being coated with a membrane according to the invention, said membrane having preferably its larger pores in contact with the inner side of the tubes and said membrane having preferably its smaller pores in contact with an effluent medium and comprising preferably an internal biofilm close to the side of the smaller pores, the intertubular space being preferably filled with a nutrient medium, the thickness of the carbon tubes being

- such that the bacteria can be immobilized in the membranes by filtration of a suspension of bacteria substantially along the outer surface of the tubes, and

- such that the nutrient medium can diffuse from the outer surface of the tubes to the inner surface of the tubes coated with the abovesaid membranes,

the thickness of the carbon tubes being preferably of about 1 to about 3 mm and of the polyester tubes being preferably of about 100 to about 500 μ.

When xenobiotics must be degraded by specialized bacteria immobilized in the membranes of flat sheet reactor, tubular membrane reactor or continuous tubular membrane reactor, cell release needs to be reduced by a very low minimum).

The reactors of the invention can be used very well for the introduction of degradation of recalcitrant molecules by the principle of cometabolism. The advantage of this invention is that low amounts of substrate can be used for induction of cometabolism, compared to the stirred tank reactors where sometimes until 7 times higher substrate concentrations than the pollutant concentration are necessary.

The flat sheet reactor (FSR), continuous flat sheet reactor (CFSR), tubular membrane reactor (TMR) and continuous tubular membrane reactor (CTMR) can be used for the degradation of xenobiotic organic compounds, thanks to the immobilization of specialized xenobiotic organic compound degrading microorganisms. At the nutrient side, the bacteria can be provided with essential elements with or without some substrate in the use of very low pollutant concentrations, or in the case of cometabolism.

This system is then able to degrade the xenobiotics, such as chlorobiphenyl compounds, completely into water, $CO_2$ and NaCl (mineralization) if the right bacteria are used and if an appropriate substrate is used.

Examples of substrates are lactic acid, aromatic compounds, chloroaromatic compounds and polyaromatic compounds.

The invention also relates to a reactor wherein a recuperation column is integrated downstream of reactor, said column being such that it enables to recover metal which has been precipitated together with bacteria involved in said precipitation, said column being preferably filled with a material liable to adsorb metal and bacteria, such as glass beads, glass wool, glass powder or other form of silicate like sand particles.

In order to recover the biologically precipitated metals, a recuperation column can be integrated in the circuit downstream of the FSR (Flat Sheet Reactor), CFSR (continuous flat sheet reaction), or TMR (Tubular Membrane Reactor), or CTMR.

Bacteria are released slowly from the reactor into the effluent, where they interact with the metals around them and afterwards they must be removed from the suspension. For this a column, preferentially filled with glass beads, glass wool or glass powder, or other silicate like sand particles, is installed downstream of the reactor. The bacteria together with the crystallized metals can bind to the glass beads, the glass wool or glass powder, or other materials of the column. When the column is completely filled and saturated with metals, she can be replaced by another one without causing some problems at the level of the membrane in the reactor. The metals can be eluted from the column by acids (this eluate can be used in electrolyses), and the column, regenerated in this way, can be reused.

The invention also relates to a process for precipitating metals or degrading xenobiotic organic compounds wherein a nutrient is administered into one of the chambers of the reactor according to the invention, and effluent to be treated containing metals or xenobiotic organic compounds are circulated in the other chamber of said reactor and wherein the bacteria in and on the membrane induce

- either precipitation and crystallization processes which results in the settling of the metals,
- or mineralization of xenobiotic organic compounds, for instance by co-metabolism of said xenobiotic compounds in the presence of a substrate, which results in degradation of said xenobiotic organic compounds into water, $CO_2$ and a mineral salt originating from the inorganic ions of said xenobiotic organic compounds and of said substrate,

said process for precipitating metals or degrading xenobiotics being carried out continuously or in batch.

The invention also relates to a process for degrading xenobiotic organic compounds,

wherein a substrate for induction of co-metabolism is used for the mineralization of xenobiotics by the bacteria into water, $CO_2$ and either inorganic salts originating from inorganic ions of said xenobiotic organic compounds and of said substrate or gaz, or wherein no substrate is used and xenobiotic organic compounds in the solutions to be treated are mineralized into water, $CO_2$ and possibly $Cl^-$ (in the case of chlormated compounds) by the bacteria.

FIGURES

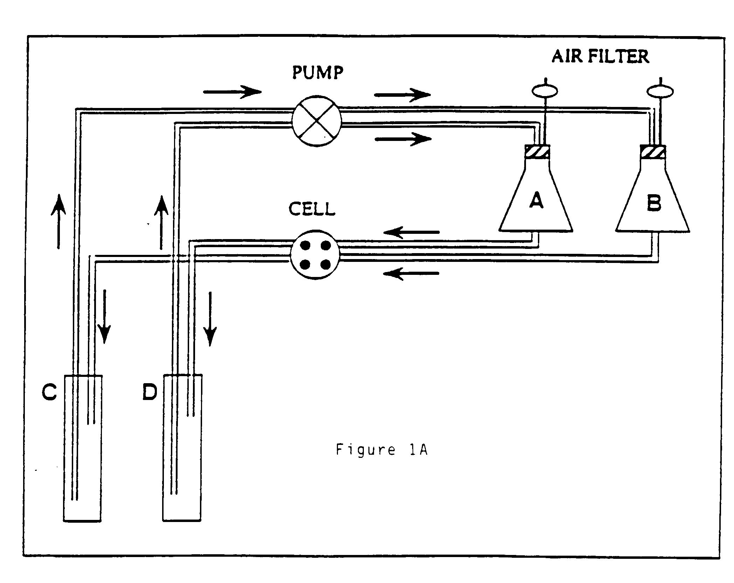

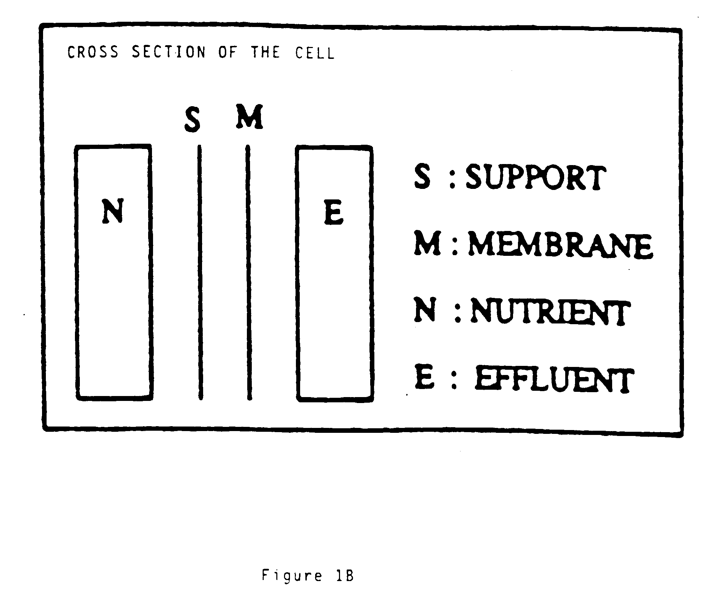

Figure 1A represents a reactor of the invention.
This reactor consists of two chambers with each an inlet and outlet and separated by each other by a membrane and a supporting frame. In one chamber nutrient solution is pumped during reactor use and at the other side a synthetic or a real effluent solution is pumped. With this system a reduction of nutrients can be obtained. The bacteria are fed at the membrane in the biofilm by nutrients (A) coming from the nutrient side and induce in that way the precipitation of the metals. Moderate amounts of nutrients (P, C, N) must be added to the effluent stream. At the effluent side, the immobilized bacteria remove the heavy metals from the effluent (B). The siphons C and D are used to keep the pressure difference over the membrane at zero.
Figure 1B represents a cross section of the reactor, represented on Figure 1A in which :

- M represents the membrane with immobilized bacteria therein and/or thereon,
- S represents the support,
- N represents the nutrient medium,
- E represents the effluent.

- Figure 2A and Figure 2B correspond to Figures 1A and 1B in which, downstream of the reactor, a recuperation column (F) is installed.

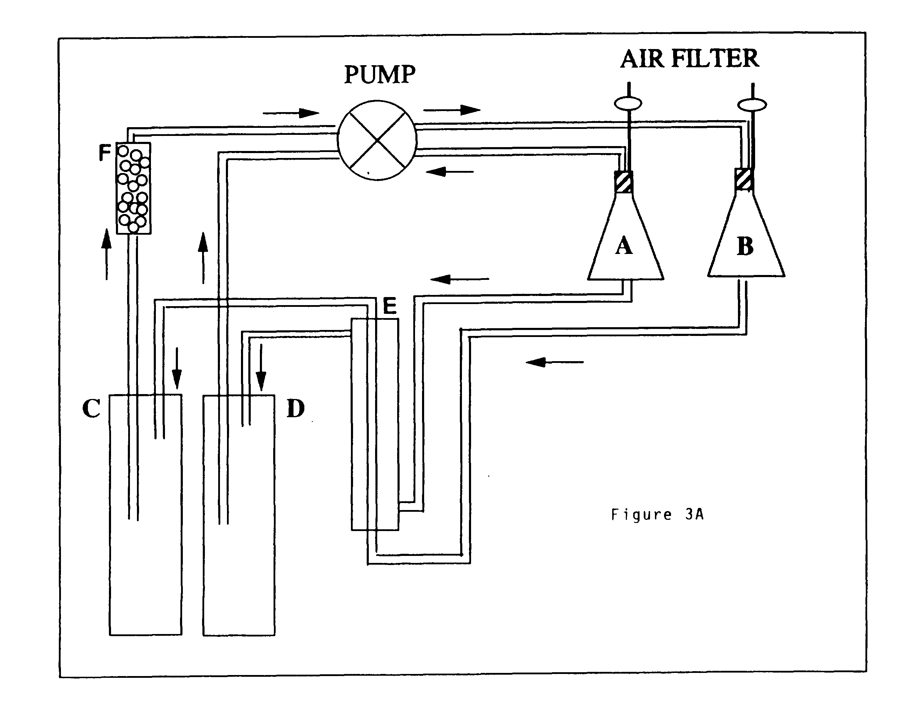

- Figure 3A represents a Tubular Membrane Reactor (TMR) . This consists of a tubular membrane (E) connected at the outer shell side with the nutrient (A) and at the inner tubular membrane side to the effluent (B). The siphons (C) and (D) regulate the pressure difference. Downstream of the reactor a recuperation column (F) is installed.

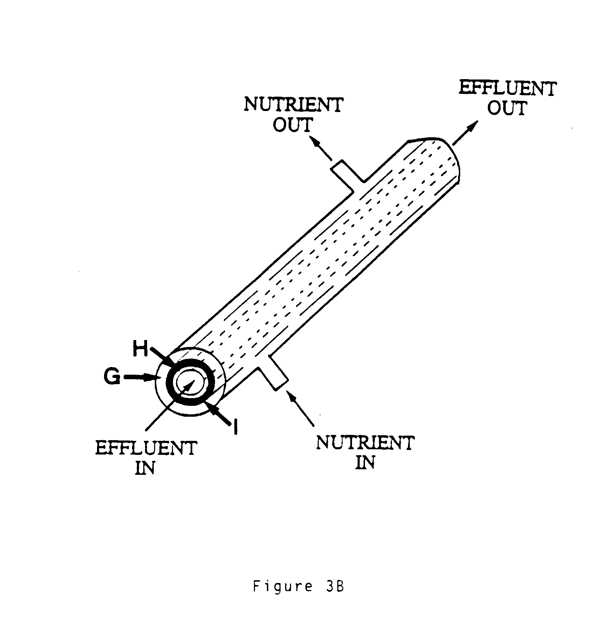

- Figure 3B represents the tubular reactor (E). It shows the composite membrane (H) at the inner side of a carbon or polyester support (I). The effluent flows through the inner tubular space (J). The nutrient through the outer tubular space (G).

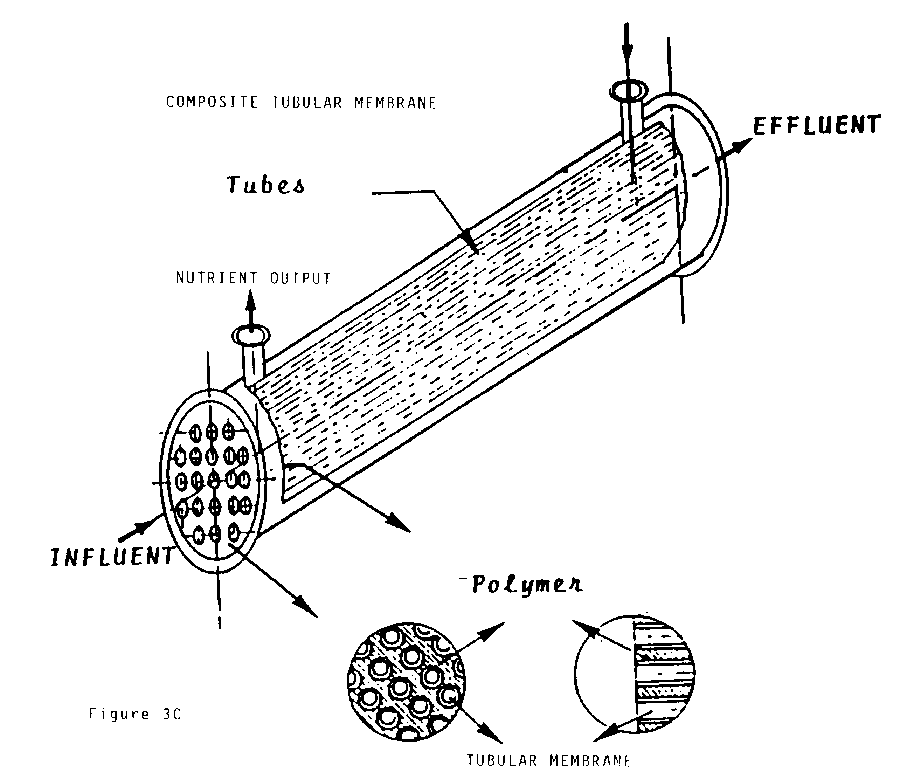

- Figure 3C represents a general view of a multipipe tubular membrane reactor.

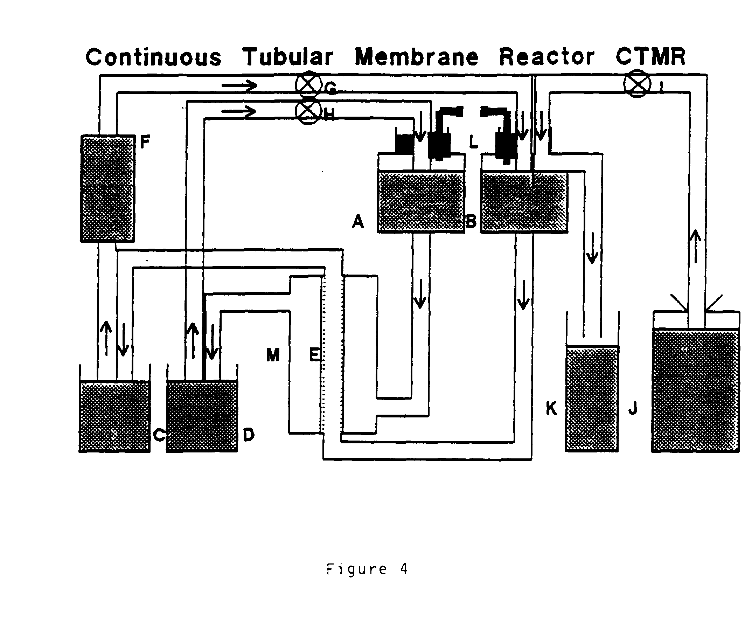

- Figure 4 represents a continuous tubular membrane reactor. The system consists of an input (J), with metal contaminated effluent, an output (K), a nutrient vessel (A) and an effluent vessel (B) each foreseen with a filter (L). Further pump (I) pumps the effluent to vessel (B), from these it is pumped by pump (G) through the TMR (M) with immobilized bacteria in the membrane (E) into siphon (C) over a glass bead column (F) again into (B). Nutrient is pumped by pump (H) from (A) to through the outer tubular space of (M) into siphon (D) back to (A).

EXAMPLE 1 : Removal of heavy metals :

The Alcaligenes eutrophus strain CH34 can be used for induction of precipitation and crystallisation of heavy metals like cadmium, lead, zinc, nickel and copper.
At the nutrient side a carbon source (0.1% to 0.8% lactate), phosphate (from 0.1 to 1.0 mM) and ammonia (from 0.5 to 5 mM) are administered to keep the bacteria efficient for the precipitation process.
At the effluent side, water solutions containing cadmium (from 5 to 250 ppm), nickel (from 20 to 100 ppm), zinc (from 20 to 600 ppm), copper (from 20 to 60 ppm) and lead (from 5 to 250 ppm) can be circulated.
The bacteria in the membrane will induce precipitation and crystallization processes at the membrane which results in a settling of these metal precipitates on the membranes or on the walls of the recirculation tank. The metals can be removed from the walls by treatment with a 1N HCl solution.
More precisely, a membrane of a surface of 10 $cm^2$ is used. Said membrane is a polysulfonic membrane (18% of polysulfone) and contains 82% of $ZrO_2$.
It has a porosity of 70%, the size of the pores is from about 1 to about 2 $\mu$ and it has a thickness of 130 $\mu$ and one of its sides is a "skin side".
The immobilization of the bacteria is carried out with 100 ml of solution containing $10^8$ bacteria/ml (it is a dilution by 10 of an overnight culture) submitted to a tangential filtration which lasts 4 hours. The colonization is carried out for 4 days in the presence of a nutrient medium containing 0.2% of lactate, 1 mM of $Na_2HPO_4$, 5 mM of $NH_4OH$.
The pH of the effluent to be treated is of 7.8.
The flow rate of the nutrient medium is of 23 ml/mn and the flow rate of the effluent medium to be treated is of 23 ml/mn.
The treatment of the effluent lasts 36 hours.
With a solution containing 224 ppm of cadmium the yield of removal of cadmium is of about 98%.

EXAMPLE 2 : Degradation of PCB :

In the following example, the conditions used, except for the strain, are the same ones as the ones described in example 1.

However, the pH of the effluent to be treated is between 6 and 8.

The Alcaligenes eutrophus strain A5 can be used for cometabolism of chlorinated biphenyl molecules.

At the nutrient side a cometabolizing carbon source (0.1 - 0.2%) is administered to keep the bacteria catabolizing (for instance biphenyl for the degradation of PCB). Also some phosphate (from 0.1 to 1.0 mM), and ammonia (from 0.5 to 5 mM) are fed to the immobilized bacteria. At the effluent side a water suspension containing some chlorinated biphenyls (from 0 to 2000 ppm) can be circulated. The biphenyls can bind easily to the hydrophobic membrane and there they will be catabolized by the immobilized bacteria.

In the case of A5 only mono-, di- and trichlorobiphenyls can be degraded.

EXAMPLE 3 : Synthesis of a melanine like polymer :

In the following example, the conditions are the same as the ones described in example 1, except for the pH of the process solution which is from about 6 to about 8.

Some spontaneous mutants of Alcaligenes eutrophus CH34 (available to the man skilled in the art) can transform compounds like tyrosine and meta hydroxylated aromatics into black colored melanine like polymers.

At the nutrient side a mineral medium with gluconate (0.2%) as carbon source and 0.1% of tyrosine (the compound to be transformed) are administered (tyrosine can also be fed at the process solution side).

At the effluent side the end product polymer will appear and can be recovered without contamination of the original compound (e.g. tyrosine).

EXAMPLE 4 : Continuous tubular membrane reactor (CTMR)

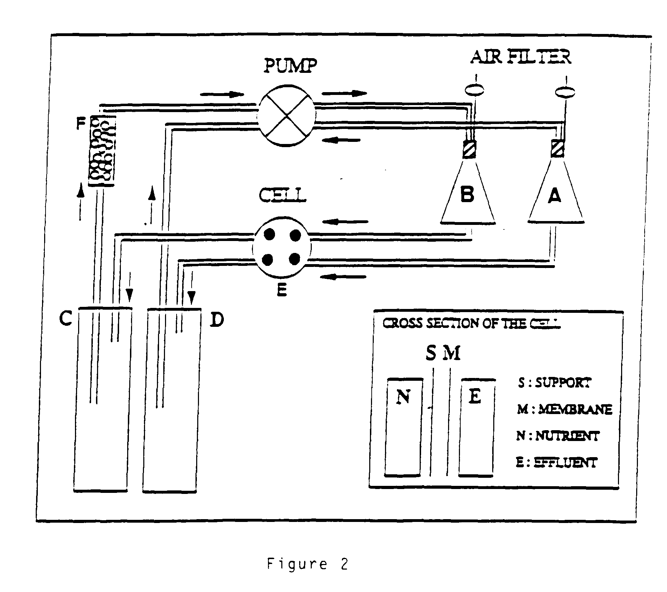

Figure 2 presents a Continuous Tubular Membrane Reactor (CTMR).

The system consists of an input (J), with metal contaminated effluent, an output (K), a nutrient vessel (A) and an effluent vessel (B) each foreseen with a filter (L). Further pump (I) pumps the effluent to vessel (B), from these it is pumped by pump (G) through the TMR (M) with immobilized bacteria in the membrane (E) into siphon (C) over a glass bead column (F) again into (B). Nutrient is pumped by pump (H) from (A) through the outer tubular space of (M) into siphon (D) back to A.

With this system 1 liter effluent with 125 ppm Cd can be treated in minor than 20 hours to below 1 ppm Cd with a membrane surface of 16 cm$^2$. The same is true for the reduction of 70 ppm Zn to below 1 ppm Zn. The lactate consumption with this system is 0.3 g lactate/l effluent.

At the membrane a minimum flow rate of between 100 and 200 ml/mn is necessary.

EXAMPLE 5 : The use of a TMR for degradation of organic compounds

In a FSR, 500 ml of a solution containing 0.8% lactic acid was treated by immobilized A. eutrophus CH34. The degradation of lactic acid was measured spectrophotometrically.

A degradation rate of 1 mg lactic acid/liter.hour.cm$^2$ membrane surface or 1 ppm lactic acid/hour.cm$^2$ membrane surface was obtained in a real crude first assay.

Higher rates can probably be obtained in a TMR and a CTMR.

The example shows the utility of the reactor system for degradation of small amounts (ppm to ppb level) of organic compounds in liquid effluents.

EXAMPLE 6 : Comparison between polysulfone and composite (polysulfone + ZrO$^2$) membrane

Two FSR reactors were run with 1.0 mM Cd in the effluent stream. In one reactor, a composite Zirfon membrane (Zirfon = polysulfone + ZrO$_2$) was used and in the other a commerical polysulfone membrane.

The removal of Cd was measured in function of time. Two results were obtained :

1. Metal diffusion through the polysulfone membrane was higher than through the Zirfon membrane (3 times higher), which implies that a substantial amount of metal can be found in the nutrient, which is something to be avoided as it is a contamination of the nutrient.

2. Metal removal was faster with the Zirfon membrane than with the polysulfone membrane (depending on the Zirfon membrane that is used, it can go up to 4 times faster). With Zirfon membranes with no cell release, only a slight difference could be seen with a polysulfone membrane (due to the high impact of the released cells in the metal removal process).

**Claims**

**1.** A membrane with immobilized microorganisms thereon and/or therein, characterized in that

- said microorganisms are alive or in a viable form and are liable to precipitate one or several metals, when they are in the presence of said metal and/or to degrade xenobiotic organic compounds, when they are in the presence of said compounds, and in that

- said membrane is of a porous material, being either an inorganic oxide or a composite material containing an inorganic oxide, the membrane parameters being such that the microorganisms can settle in the pores, which are communicating between themselves, and which allow an appropriate colonization of said membrane by said microorganisms, and that their release is less than $10^4$ microorganisms/ml/h, preferably less than $10^2$ microorganisms/ml/h.

2. The membrane according to claim 1, wherein the microorganisms are selected from the group consisting of fungi, yeast, algae and preferably bacteria.

3. The membrane with immobilized microorganisms, particularly bacteria, according to claims 1 or 2, wherein the pores of the membranes have an average size of 1 μm to 10 μm, preferably 1 μm to 3 μm.

4. The membrane with immobilized microorganisms, particularly bacteria, according to any of the claims 1 to 3, wherein the porosity lies between 50% and 80%, preferably between 65% and 75%.

5. The membrane with immobilized microorganisms, particularly bacteria, according to any of the claims 1 to 4, wherein the thickness of the membrane lies between 50 μm and 700 μm, preferably between 70 μm and 500 μm and more advantageously between 250 μm and 500 μm.

6. The membrane with immobilized microorganisms, particularly bacteria, according to any of the claims 1 to 5, wherein one of the sides of the membrane forms a skin having pores, the size of which prevents the microorganisms from being released, the size of these pores being smaller than 0.5 μm.

7. The membrane with immobilized microorganisms according to any of the claims 1 to 6, wherein said microorganisms are bacteria and wherein on one of its sides, said bacteria are in the form of a biofilm having a thickness of between 1 μm and 50 μm, preferably between 10 μm and 20 μm, and internally said bacteria form a colonizing front, around single dispersed bacteria in the membrane.

8. The membrane with immobilized microorganisms, particularly bacteria, according to any of the claims 1 to 7, wherein the microorganisms are settled in the pores of said membrane, to form a colonizing front on a thickness which varies from 10 μm to the totality of the thickness of said membrane, prefera-

bly from 50 μm to the totality of the thickness of said membrane.

9. The membrane with immobilized microorganisms, particularly bacteria, according to claim 1, wherein said membrane is a flat membrane or a tubular membrane with an inner diameter preferably higher than 2 mm, with or without a support.

10. The membrane with immobilized microorganisms, particularly bacteria, according to any of the claims 1 to 9, wherein said membrane is flat and has an external side with a larger pore side with a biofilm of microorganisms on said external side.

11. The membrane with immobilized microorganisms, particularly bacteria, according to any of the claims 1 to 9, wherein said membrane is tubular and a colonizing front of bacteria is close to the internal side of said membrane which has the smaller pore size.

12. The membrane with immobilized microorganisms, particularly bacteria, according to any of the claims 1 to 9 or 11, wherein said membrane coats the inner surface of a tube, preferably a carbon tube or a polyester tube, the larger size pores of said membrane being in contact with the inner surface of said tube and the colonizing front being inside the membrane, preferably close to the skin side.

13. The membrane with immobilized microorganisms, particularly bacteria, according to any of the claims 1 to 12, wherein said membrane is made of polysulfone comprising an inorganic material, such as polysulfone comprising $ZrO_2$ or is made of $ZrO_2$.

14. The membrane with immobilized bacteria according to anyone of claims 1 to 13, wherein the pressure difference between each side of the membrane is about 0.

15. A process for preparing a membrane according to any of the claims 1 to 14, comprising the step of:

- filtrating a suspension of microorganisms, said suspension containing an amount of microorganisms such that the membrane is not clogged, said suspension containing between $10^7$ and $10^9$ microorganisms/ml, preferably $10^8$ microorganisms/ml, the filtration being carried out through the membrane, preferably in a tangential manner with respect to the surface of the membrane, in order to immobilize the microorganisms in and/or on the membrane.

16. A reactor comprising a cell containing:

- the membrane according to any of the claims 1 to 14,

- optionally a support,
- two parts with each an inlet and an outlet and separated by a membrane, one part being in contact with one side of the membrane and containing a nutrient medium to enable the life and growth of the microorganisms, and the other part being in contact with the other side of the membrane and containing an effluent to be treated.

17. The reactor according to claim 16, comprising

- a flat membrane according to any of the claims 1 to 10, 13 or 14, said membrane separating two chambers with each an inlet and an outlet, one of said chambers containing a nutrient medium and the other chamber containing an effluent to be treated, the chamber containing the nutrient medium being preferably in contact with the side of the membrane with the smaller pores and the chamber containing the effluent medium being preferably in contact with the side of the membrane with the larger pores, and more preferably with a biofilm of microorganisms located externally on the side of the membrane with larger pores.

18. The reactor according to claim 16, comprising

- a tubular recipient, said recipient containing hollow supporting tubes of a porous material, said supporting tubes being preferably carbon tubes or polyester tubes, the inner surface or the outer surface of the supporting tubes being coated with a membrane according to any of the claims 1 to 9 or 11 to 14, said membrane being in contact with the effluent to be treated, the thickness of said carbon tubes being
- such that the microorganisms can be immobilized in the membranes by filtration of a suspension of microorganisms substantially along the outer or the inner surface of the tubes, and
- such that the nutrient medium which is either in the tube or in the intertubular space can diffuse from the outer or the inner surface of the tube respectively to the inner or to the outer surface of the tube coated with said membrane.

19. The reactor according to claim 16, comprising:

- a tubular recipient, said recipient containing hollow supporting tubes of a porous material, said supporting tubes being carbon tubes or polyester tubes, the inner surface of the supporting tubes being coated with a membrane according to any of the claims 1 to 14, said membrane having preferably its larger pores in contact with the inner side of the supporting tubes and said membrane having preferably its smaller pores in contact with an effluent medium and comprising an internal colonizing front, preferably close to the side of the smaller pores, the intertubular space being preferably filled with a nutrient medium, the thickness of the carbon tubes being

- such that the microorganisms are immobilized in the membranes by filtration of a suspension of microorganisms substantially along the outer surface of the tubes, and
- such that the nutrient medium can diffuse from the outer surface of the tubes to the inner surface of the tubes coated with said membrane, the thickness of the carbon tubes being preferably comprised between 1 mm and 3 mm and of the polyester tubes being preferably comprised between 100 μm and 500 μm.

20. The reactor according to anyone of claims 16 to 19, wherein a recuperation column is integrated downstream of said reactor, said column being such that it enables to recover metal which has been precipitated together with bacteria involved in said precipitation, said column being preferably filled with a material liable to adsorb metal and bacteria, such as glass beads, glass powder, glass wool or any form of silicate like sand particles.

21. A process for precipitating metals or degrading xenobiotic organic compounds wherein a nutrient is administered into one of the chambers of the reactor according to anyone of claims 17 to 20, and effluents to be treated containing metals or xenobiotic organic compounds are circulated in the other chamber of said reactor and wherein the bacteria in and on the membrane induce

- either precipitation and crystallization processes which results in the settling of the metals,
- or mineralization of xenobiotic organic compounds, for instance by co-metabolism of said xenobiotic compounds in the presence of a substrate, which results in degradation of said xenobiotic organic compounds into water, $CO_2$ and a mineral salt originating from the inorganic ions of said xenobiotic organic compounds, and from said substrate, said process for precipitating metals or degrading xenobiotics being carried out continuously or in batch.

22. The process for degrading xenobiotic organic compounds according to claim 21, wherein a substrate for induction of co-metabolism is used for the mineralization of xenobiotics by the bacteria into water, $CO_2$ and either inorganic salts originating from inorganic ions of said xenobiotic organic compounds and of said substrate or gaz, or wherein no substrate is used and xenobiotic organic compounds in the solutions to be treated are mineralized into water, $CO_2$ and possibly Cl⁻ by the bacteria.

## Patentansprüche

1. Membrane mit darauf und/oder darin immobilisierten Mikroorganismen, dadurch gekennzeichnet, dass

   - diese Mikroorganismen leben oder in einer lebensfähigen Form vorliegen und dazu neigen ein oder mehrere Metalle auszufällen, wenn sie sich in Gegenwart dieses Metalls befinden und/oder dazu neigen xenobiotische organische Verbindungen abzubauen, wenn sie sich in Gegenwart solcher Verbindungen befinden, und dadurch, dass

   - diese Membran aus einem porösen Material besteht, das entweder ein anorganisches Oxid oder ein Verbundmaterial ist, das ein anorganisches Oxid enthält, wobei die Membranparameter derart sind, dass die Mikroorganismen sich in den Poren festsetzen können, welche miteinander in Verbindung stehen, und welche eine angemessene Besiedelung dieser Membran durch die Mikroorganismen erlauben, und dass deren Freigabe sich auf weniger als $10^4$ Mikroorganismen/ml/h, vorzugsweise weniger als $10^2$ Mikroorganismen/ml/h beläuft.

2. Membran gemäß Anspruch 1, in welcher die Mikroorganismen ausgewählt werden aus der Gruppe, die aus Pilzen, Hefen, Algen und vorzugsweise Bakterien besteht.

3. Membran mit immobilisierten Mikroorganismen, insbesondere Bakterien, gemäß den Ansprüchen 1 oder 2, in welcher die Poren der Membranen eine mittlere Größe von 1 μm bis 10 μm, vorzugsweise von 1 μm bis 3 μm besitzen.

4. Membran mit immobilisierten Mikroorganismen, insbesondere Bakterien, gemäß irgendeinem der Ansprüche 1 bis 3, in welcher die Porosität zwischen 50% und 80%, vorzugsweise zwischen 65% und 75% liegt.

5. Membran mit immobilisierten Mikroorganismen, insbesondere Bakterien, gemäß irgendeinem der Ansprüche 1 bis 4, in welcher die Dicke der Membran zwischen 50 μm und 700 μm, vorzugsweise zwischen 70 μm und 500 μm und in besonders vorteilhafter Weise zwischen 250 μm und 500 μm liegt.

6. Membran mit immobilisierten Mikroorganismen, insbesondere Bakterien, gemäß irgendeinem der Ansprüche 1 bis 5, in welcher eine der Seiten der Membran eine Haut bildet, die Poren besitzt deren Größe die Mikroorganismen davor schützt freigesetzt zu werden, wobei die Größe dieser Poren kleiner ist als 0,5 μm.

7. Membran mit immobilisierten Mikroorganismen gemäß irgendeinem der Ansprüche 1 bis 6, in welcher die Mikroorganismen Bakterien sind und in welcher auf einer ihrer Seiten diese Bakterien in der Form eines Biofilms mit einer Dicke zwischen 1 μm und 50 μm, vorzugsweise zwischen 10 μm und 20 μm vorliegen und die Bakterien im Inneren eine Besiedlungsfront um einzelne dispergierte Bakterien in der Membran bilden.

8. Membran mit immobilisierten Mikroorganismen, insbesondere Bakterien, gemäß irgendeinem der Ansprüche 1 bis 7, in welcher die Mikroorganismen in den Poren der besagten Membran angesiedelt sind, um eine Besiedlungsfront von einer Dicke zu bilden, die von 10 μm bis zur Gesamtdicke dieser Membran variiert, vorzugsweise von 50 μm bis zur Gesamtdicke dieser Membran.

9. Membran mit immobilisierten Mikroorganismen, insbesondere Bakterien, gemäß Anspruch 1, in welcher die Membran eine flache Membran oder eine röhrenförmige Membran ist mit einem Innendurchmesser von vorzugsweise mehr als 2 mm, mit oder ohne einen Träger.

10. Membran mit immobilisierten Mikroorganismen, insbesondere Bakterien, gemäß irgendeinem der Ansprüche 1 bis 9, in welcher die Membran flach ist und eine äußere Seite mit einer ausgedehnteren Porengröße mit einem Biofilm an Mikroorganismen auf dieser äußeren Seite aufweist.

11. Membran mit immobilisierten Mikroorganismen, insbesondere Bakterien, gemäß irgendeinem der Ansprüche 1 bis 9, in welcher die Membran röhrenförmig ist und sich eine Besiedlungsfront an Bakterien in der Nähe der inneren Seite dieser Membran, welche die kleinere Porengröße besitzt, befindet.

12. Membran mit immobilisierten Mikroorganismen, insbesondere Bakterien, gemäß irgendeinem der Ansprüche 1 bis 9 oder 11, in welcher die Membran die innere Oberfläche eine Röhre bedeckt, vorzugsweise einer Kohlenstoffröhre oder einer Polyesterröhre, wobei die größeren Poren dieser Membran in Berührung mit der inneren Oberfläche dieser Röhre stehen und sich die Besiedlungsfront innerhalb der Membran befindet, vorzugsweise in der Nähe der Hautseite.

**13.** Membran mit immobilisierten Mikroorganismen, insbesondere Bakterien, gemäß irgendeinem der Ansprüche 1 bis 12, in welcher diese Membran aus Polysulfon hergestellt ist, das ein anorganisches Material aufweist, wie z.B. ein $ZrO_2$ enthaltendes Polysulfon, oder aus $ZrO_2$ hergestellt ist.

**14.** Membran mit immobilisierten Bakterien gemäß irgendeinem der Ansprüche 1 bis 13, in welcher der Druckunterschied zwischen einer jeden Seite der Membran etwa 0 beträgt.

**15.** Verfahren zum Herstellen einer Membran gemäß irgendeinem der Ansprüche 1 bis 14, das den folgenden Schritt enthält:

- Filtrieren einer Suspension von Mikroorganismen, wobei diese Suspension eine solche Menge an Mikroorganismen enthält, dass die Membran nicht verstopft wird, wobei diese Suspension zwischen $10^7$ und $10^9$ Mikroorganismen/ml, vorzugsweise $10^8$ Mikroorganismen/ml enthält, wobei die Filtration durch die Membran vorgenommen wird, vorzugsweise in einer tangentialen Art und Weise in Bezug auf die Oberfläche der Membran, um die Mikroorganismen in und/oder auf der Membran zu immobilisieren.

**16.** Reaktor mit einer Zelle, welcher enthält:

- die Membran gemäß irgendeinem der Ansprüche 1 bis 14,

- wahlweise einen Träger,

- zwei Teile, ein jeder mit einem Einlass und einem Auslass und durch eine Membran getrennt, wobei ein Teil in Berührung mit einer Seite der Membran steht und ein Nährmedium enthält, um das Leben und das Wachstum der Mikroorganismen zu ermöglichen, und der andere Teil in Berührung mit der anderen Seite der Membran steht und ein Abwasser enthält, das behandelt werden soll.

**17.** Reaktor gemäß Anspruch 16, welcher enthält:

- eine flache Membran gemäß irgendeinem der Ansprüche 1 bis 10, 13 oder 14, wobei diese Membran zwei Kammern mit jeweils einem Einlass und einem Auslass voneinander trennt, wobei eine dieser Kammern ein Nährmedium und die andere Kammer ein zu behandelndes Abwasser enthält, wobei die das Nährmedium enthaltende Kammer vorzugsweise in Berührung mit der Seite der Membran steht, welche die kleineren Poren besitzt und die das Abwasser enthaltende Kammer vorzugsweise in Berührung mit der Seite der Membran steht, welche die größeren Poren besitzt, und noch bevorzugter mit einem Biofilm an Mikroorganismen, der sich außen auf der Seite der Membran mit den großen Poren befindet.

**18.** Reaktor gemäß Anspruch 16, welcher enthält:

- einen röhrenförmiger Empfänger, wobei dieser Empfänger hohle Trägerröhren aus einem porösen Material enthält, wobei diese Trägerröhren vorzugsweise aus Kohlenstoffröhren oder Polyesterröhren bestehen, wobei die innere Oberfläche oder die äußere Oberfläche der Trägerröhren mit einer Membran gemäß irgendeinem der Ansprüche 1 bis 9 oder 11 bis 14 beschichtet ist, wobei die Membran in Kontakt mit dem zu behandelnden Abwasser steht, und die Dicke dieser Kohlenstoffröhren derart ist,

- dass die Mikroorganismen in den Membranen durch Filtration einer Suspension der Mikroorganismen im Wesentlichen entlang der äußeren oder inneren Oberfläche der Röhren immobilisiert werden können, und

- dass das Nährmedium, das sich entweder in der Röhre oder in den Raum zwischen den Röhren befindet, von der äußeren bzw. der inneren Oberfläche der Röhre zu der inneren bzw. der äußeren Oberfläche der Röhre, die mit dieser Membran beschichtet ist, diffundieren kann.

**19.** Reaktor gemäß Anspruch 16, welcher enthält:

- einen röhrenförmiger Empfänger, wobei dieser Empfänger hohle Trägerröhren aus einem porösen Material enthält, wobei diese Trägerröhren vorzugsweise aus Kohlenstoffröhren oder Polyesterröhren bestehen, wobei die innere Oberfläche der Trägerröhren mit einer Membran gemäß irgendeinem der Ansprüche 1 bis 14 beschichtet ist, wobei die Membran vorzugsweise mit ihren größeren Poren in Kontakt mit der inneren Seite der Trägerröhren steht, und wobei die Membran vorzugsweise mit ihren kleineren Poren in Kontakt mit dem Abwassermedium steht und eine innere Besiedelungsfront aufweist, vorzugsweise in der Nähe der Seite mit den kleineren Poren, wobei der Raum zwischen den Röhren vorzugsweise gefüllt ist mit einem Nährmedium, wobei die Dicke dieser Kohlenstoffröhren derart ist,

- dass die Mikroorganismen in den Membranen durch Filtration einer Suspension der Mikroorganismen im Wesentlichen entlang der äußeren Oberfläche der Röhren immobilisiert werden, und

- das Nährmedium von der äußeren Oberfläche der Röhren zu der inneren Oberfläche der Röhren, die mit dieser Membran beschichtet sind, diffundieren kann, wobei die Dicke der Kohlenstoffröhren vorzugsweise zwischen 1 mm und 3 mm und diejenige der Polyesterröhren vorzugsweise zwischen 100 μm und 500 μm liegt.

20. Reaktor gemäß irgendeinem der Ansprüche 16 bis 19, in welchem eine Rückgewinnungskolonne stromabwärts des Reaktors integriert ist, wobei diese Kolonne derart ist, dass sie es ermöglicht Metall zurückzugewinnen, das zusammen mit den Bakterien ausgefällt worden ist, die in dieser Ausfällung impliziert sind, wobei die Kolonne vorzugsweise mit einem Material gefüllt ist, das dazu neigt Metall und Bakterien zu adsorbieren, wie z.B. Glasperlen, Glaspulver, Glaswolle oder jede Form von Silikat, wie Sandpartikel.

21. Verfahren zum Ausfällen von Metallen oder von abbauenden xenobiotischen organischen Verbindungen, in welchem ein Nährmedium in eine der Kammern des Reaktors gemäß irgendeinem der Ansprüche 17 bis 20 gegeben wird, und Abwässer, die behandelt werden sollen und Metalle oder xenobiotische organische Verbindungen enthalten in der anderen Kammer dieses Reaktors umlaufen gelassen werden, und in welchem die Bakterien in und auf der Membran Auslöser sind für:

- entweder Ausfällungs- oder Kristallisationsverfahren, die zu einem Absetzen der Metalle führt,

- oder eine Mineralisierung von xenobiotischen organischen Verbindungen, zum Beispiel durch gemeinsamen Stoffwechsel dieser xenobiotischen Verbindungen in Gegenwart eines Substrates, was zu einem Abbau der xenobiotischen organischen Verbindungen zu Wasser, $CO_2$ und einem Mineralsalz führt, das aus den anorganischen Ionen dieser xenobiotischen organischen Verbindungen und dem besagten Substrat herrührt, wobei das Verfahren zur Ausfällung von Metallen oder zum Abbau von Xenobiotika kontinuierlich oder in einem chargenweisen Betrieb durchgeführt wird.

22. Verfahren zum Abbau von xenobiotischen organischen Verbindungen gemäß Anspruch 21, in welchem ein Substrat zum Auslösen des gemeinsamen Stoffwechsels verwendet wird für die Mineralisierung der Xenobiotika durch die Bakterien zu Wasser, $CO_2$ und anorganischen Salzen, die herrühren von den anorganischen Ionen dieser xenobiotischen organischen Verbindungen und aus dem besagten Substrat oder Gas, oder in welchem kein Substrat verwendet wird und die xenobiotischen organischen Verbindungen in den Lösungen, die behandelt werden sollen, zu Wasser, $CO_2$ und möglicherweise $Cl^-$ durch die Bakterien mineralisiert werden.

## Revendications

1. Membrane sur et/ou dans laquelle sont immobilisés des microorganismes, caractérisée en ce que :

- lesdits microorganismes sont vivants ou sous une forme viable et sont susceptibles de précipiter un ou plusieurs métaux, lorsqu'ils sont en présence dudit métal, et/ou de dégrader des composés organiques xénobiotiques, lorsqu'ils sont en présence desdits composés, et en ce que :

- ladite membrane est constituée d'un matériau poreux, qui est un oxyde inorganique ou un matériau composite contenant un oxyde inorganique, les paramètres de la membrane étant tels que les microorganismes puissent se déposer dans les pores, qui communiquent entre eux, et permettent une colonisation appropriée de ladite membrane par lesdits microorganismes, et en ce que leur libération est inférieure à $10^4$ microorganismes/ml/h, de préférence inférieure à $10^2$ microorganismes/ml/h.

2. Membrane selon la revendication 1, dans laquelle les microorganismes sont sélectionnés dans le groupe constitué de champignons, de levure, d'algues et de préférence de bactéries.

3. Membrane à microorganismes immobilisés, en particulier des bactéries, selon la revendication 1 ou 2, dans laquelle les pores des membranes ont une taille moyenne de 1 à 10 micromètres, de préférence de 1 à 3 micromètres.

4. Membrane à microorganismes immobilisés, en particulier des bactéries, selon l'une quelconque des revendications 1 à 3, dans laquelle la porosité se situe entre 50% et 80%, de préférence entre 65% et 75%.

5. Membrane à microorganismes immobilisés, en particulier des bactéries, selon l'une quelconque des revendications 1 à 4, dans laquelle l'épaisseur de la membrane se situe entre 50 et 700 micromètres, de préférence entre 70 et 500 micromètres et plus avantageusement entre 250 et 500 micromètres.

**6.** Membrane à microorganismes immobilisés, en particulier des bactéries, selon l'une quelconque des revendications 1 à 5, dans laquelle une des faces de la membrane forme une peau qui possède des pores dont la taille empêche la libération des microorganismes, la taille de ces pores étant inférieure à 0,5 micromètre.

**7.** Membrane à microorganismes immobilisés selon l'une quelconque des revendications 1 à 6, dans laquelle lesdits microorganismes sont des bactéries et dans laquelle, sur une de leurs faces, lesdites bactéries se présentent sous la forme d'un film biologique dont l'épaisseur se situe entre 1 et 50 micromètres, de préférence entre 10 et 20 micromètres et, à l'intérieur, lesdites bactéries forment un front de colonisation autour de bactéries individuelles dispersées dans la membrane.

**8.** Membrane à microorganismes immobilisés, en particulier des bactéries, selon l'une quelconque des revendications 1 à 7, dans laquelle les microorganismes se déposent dans les pores de ladite membrane pour former un front de colonisation sur une épaisseur variant de 10 micromètres à la totalité de l'épaisseur de ladite membrane, de préférence de 50 micromètres à la totalité de l'épaisseur de ladite membrane.

**9.** Membrane à microorganismes immobilisés, en particulier des bactéries, selon la revendication 1, dans laquelle ladite membrane est une membrane plate ou une membrane tubulaire d'un diamètre interne de préférence supérieur à 2 mm, avec ou sans support.

**10.** Membrane à microorganismes immobilisés, en particulier des bactéries, selon l'une quelconque des revendications 1 à 9, dans laquelle ladite membrane est plate et a une face externe comprenant une face à pores plus grands avec un film biologique de microorganismes sur ladite face externe.

**11.** Membrane à microorganismes immobilisés, en particulier des bactéries, selon l'une quelconque des revendications 1 à 9, dans laquelle ladite membrane est tubulaire et un front de colonisation de bactéries est proche de la face interne de ladite membrane qui a la plus faible taille de pores.

**12.** Membrane à microorganismes immobilisés, en particulier des bactéries, selon l'une quelconque des revendications 1 à 9 ou 11, dans laquelle ladite membrane recouvre la surface interne d'un tube, de préférence un tube en carbone ou en polyester, les pores de plus grande taille de ladite membrane étant en contact avec la surface interne dudit tube et le front de colonisation se trouvant à l'intérieur de la membrane, de préférence près de la face de la peau.

**13.** Membrane à microorganismes immobilisés, en particulier des bactéries, selon l'une quelconque des revendications 1 à 12, dans laquelle ladite membrane est constituée d'une polysulfone comprenant un matériau inorganique, tel qu'une polysulfone comprenant du $ZrO_2$, ou est constituée de $ZrO_2$.

**14.** Membrane à bactéries immobilisées selon l'une quelconque des revendications 1 à 13, dans laquelle la différence de pression entre chaque face de la membrane est d'environ 0.

**15.** Procédé de préparation d'une membrane selon l'une quelconque des revendications 1 à 14, comprenant l'étape consistant :

- à filtrer une suspension de microorganismes, ladite suspension contenant une quantité de microorganismes telle que la membrane n'est pas obstruée, ladite suspension contenant entre $10^7$ et $10^9$ microorganismes/ml, de préférence $10^8$ microorganismes/ml, la filtration s'effectuant à travers la membrane, de préférence de manière tangentielle par rapport à la surface de la membrane pour immobiliser les microorganismes dans et/ou sur la membrane.

**16.** Réacteur comprenant une cellule qui contient :

- la membrane selon l'une quelconque des revendications 1 à 14,

- facultativement, un support,

- deux parties qui ont chacune une entrée et une sortie et qui sont séparées par une membrane, une partie étant en contact avec une face de la membrane et contenant un milieu nutritif pour permettre la vie et la croissance des microorganismes et l'autre partie étant en contact avec l'autre face de la membrane et contenant un effluent à traiter.

**17.** Réacteur selon la revendication 16, comprenant :

- une membrane plate selon l'une quelconque des revendications 1 à 10, 13 ou 14, ladite membrane séparant deux chambres ayant chacune une entrée et une sortie, une desdites chambres contenant un milieu nutritif et l'autre chambre contenant un effluent à traiter, la chambre contenant le milieu nutritif étant de préférence en contact avec la face de la membrane ayant les plus petits pores et la chambre contenant l'effluent étant de préférence en contact avec la face de la membrane ayant les plus gros pores, mieux encore avec un film biologique de microorganismes agencé à l'extérieur sur la face de la membrane à plus gros pores.

**18.** Réacteur selon la revendication 16, comprenant :

- un récipient tubulaire, ledit récipient contenant des tubes de support creux en matériau poreux, lesdits tubes de support étant de préférence des tubes en carbone ou en polyester, la surface interne ou la surface externe des tubes de support étant revêtue d'une membrane selon l'une quelconque des revendications 1 à 9 ou 11 à 14, ladite membrane étant en contact avec l'effluent à traiter, l'épaisseur desdits tubes en carbone étant telle :

- que les microorganismes puissent être immobilisés dans les membranes par filtration d'une suspension de microorganismes sensiblement le long de la surface externe ou interne des tubes, et

- que le milieu nutritif qui est dans le tube ou dans l'espace intertubulaire puisse diffuser de la surface externe ou de la surface interne du tube respectivement à la surface interne ou à la surface externe du tube revêtue de ladite membrane.

**19.** Réacteur selon la revendication 16, comprenant :

- un récipient tubulaire, ledit récipient contenant des tubes de support creux en matériau poreux, lesdits tubes de support étant des tubes en carbone ou en polyester, la surface interne des tubes de support étant revêtue d'une membrane selon l'une quelconque des revendications 1 à 14, ladite membrane ayant de préférence ses plus gros pores en contact avec la face interne des tubes de support et ladite membrane ayant de préférence ses plus petits pores en contact avec un effluent et comprenant un front de colonisation interne, de préférence proche de la face à plus petits pores, l'espace intertubulaire étant de préférence rempli d'un milieu nutritif, l'épaisseur des tubes en carbone étant telle :

- que les microorganismes soient immobilisés dans les membranes par filtration d'une suspension des microorganismes sensiblement le long de la surface externe des tubes, et

- que le milieu nutritif puisse diffuser de la surface externe des tubes vers la surface interne des tubes revêtue de ladite membrane, l'épaisseur des tubes en carbone étant de préférence comprise entre 1 et 3 mm et celle des tubes en polyester étant de préférence comprise entre 100 et 500 micromètres.

**20.** Réacteur selon l'une quelconque des revendications 16 à 19, dans lequel une colonne de récupération est intégrée en aval dudit réacteur, ladite colonne étant telle qu'elle permette de récupérer le métal qui a précipité avec les bactéries impliquées dans ladite précipitation, ladite colonne étant de préférence remplie d'un matériau susceptible d'adsorber le métal et les bactéries, notamment des billes de verre, du verre pulvérisé, de la laine de verre et une forme quelconque de silicate comme des particules de sable.

**21.** Procédé de précipitation de métaux ou de dégradation de composés organiques xénobiotiques, dans lequel une substance nutritive est administrée à une des chambres du réacteur selon l'une quelconque des revendications 17 à 20 et les effluents à traiter contenant des métaux ou des composés organiques xénobiotiques circulent dans l'autre chambre dudit réacteur, et dans lequel les bactéries dans et sur la membrane induisent :

- des processus de précipitation et de cristallisation qui ont pour résultat le dépôt des métaux, ou

- la minéralisation de composés organiques xénobiotiques, par exemple par co-métabolisme desdits composés xénobiotiques en présence d'un substrat, qui entraîne la dégradation desdits composés organiques xénobiotiques dans de l'eau, du $CO_2$ et un sel minéral provenant des ions inorganiques desdits composés organiques xénobiotiques et dudit substrat, ledit processus de précipitation des métaux ou de dégradation des xénobiotiques s'effectuant en continu ou en discontinu.

**22.** Procédé de dégradation de composés organiques xénobiotiques selon la revendication 21, dans lequel on utilise un substrat pour l'induction du co-métabolisme pour la minéralisation de xénobiotiques par les bactéries dans de l'eau, du $CO_2$ et des sels inorganiques provenant des ions inorganiques desdits composés organiques xénobiotiques et dudit substrat ou du gaz, ou dans lequel on n'utilise pas de substrat et les composés organiques xénobiotiques dans les solutions à traiter sont minéralisés dans de l'eau, du $CO_2$ et éventuellement du $Cl^-$ par les bactéries.

Figure 1A

CROSS SECTION OF THE CELL

S   M

N   E

S : SUPPORT

M : MEMBRANE

N : NUTRIENT

E : EFFLUENT

Figure 1B

Figure 2

Figure 3A

NUTRIENT
OUT

EFFLUENT
OUT

H

G

I

EFFLUENT
IN

NUTRIENT
IN

Figure 3B

COMPOSITE TUBULAR MEMBRANE

EFFLUENT

Tubes

NUTRIENT OUTPUT

INFLUENT

Polymer

Figure 3C

TUBULAR MEMBRANE

EP 0 579 630 B1

Figure 4